# EUROPEAN PATENT SPECIFICATION

(11) **EP 3 802 820 B1**
(45) Date of publication and mention of the grant of the patent: **07.09.2022**
(21) Application number: 19742258.7
(22) Date of filing: 03.06.2019
(51) Int. Cl.: C12N 15/10

(54) **METHODS FOR IDENTIFYING PROMOTERS FOR PROTEIN PRODUCTION IN YEAST**
VERFAHREN ZUR IDENTIFIZIERUNG VON PROMOTOREN ZUR PROTEINPRODUKTION IN HEFE
PROCÉDÉS D'IDENTIFICATION DE PROMOTEURS POUR LA PRODUCTION DE PROTÉINES DANS LA LEVURE

(30) Priority: 07.06.2018 US 201862682059 P
(43) Date of publication of application: 14.04.2021
(73) Proprietor: BASF SE, 67056 Ludwigshafen am Rhein (DE)
(72) Inventor: TAN, Xuqiu, San Diego, California 92121 (US); DODGE, Corey, San Diego, California 92121 (US); WONG, Kevin Li, San Diego, California 92121 (US); MILAN, Aileen Aboy, San Diego, California 92121 (US); ORCHARD, Samantha, San Diego, California 92121 (US); CAI, Ruorong, San Diego, California 92121 (US)
(74) Representative: BASF IP Association
(86) International application number: PCT/US2019/035143
(87) International publication number: WO 2019/236452

(56) References cited:
- WO-A1-2014/008729
- US-A1- 2012 142 053
- LIN-CEREGHINO G P ET AL: "Mxr1p, a key regulator of the methanol utilization pathway and peroxisomal genes in Pichia pastoris", MOLECULAR AND CELLULAR BIOLOGY,, vol. 26, no. 3, 1 February 2006 (2006-02-01), pages 883-897, XP002438945, ISSN: 0270-7306, DOI: 10.1128/MCB.26.3.883-897.2006
- NAKANO ET AL: "Effects of methanol feeding methods on chimeric @a-amylase expression in continuous culture of Pichia pastoris", JOURNAL OF BIOSCIENCE AND BIOENGINEE, ELSEVIER, AMSTERDAM, NL, vol. 101, no. 3, 1 March 2006 (2006-03-01) , pages 227-231, XP005446006, ISSN: 1389-1723, DOI: 10.1263/JBB.101.227
- RASCHKE W C ET AL: "Inducible expression of a heterologous protein in Hansenula polymorpha using the alcohol oxidase 1 promoter of Pichia pastoris", GENE, ELSEVIER, AMSTERDAM, NL, vol. 177, no. 1, 24 October 1996 (1996-10-24), pages 163-167, XP004043391, ISSN: 0378-1119, DOI: 10.1016/0378-1119(96)00293-4
- HARTNER F S ET AL: "Regulation of methanol utilisation pathway genes in yeasts", MICROBIAL CELL FACTORIES,, vol. 5, no. 39, 14 December 2006 (2006-12-14), pages 1-21, XP002438946, ISSN: 1475-2859, DOI: 10.1186/1475-2859-5-39
- DATABASE EMBL [Online] 2 March 2011 (2011-03-02), "LUSGC1NG-RP-103_F03_26JAN2007_021 LUSGC1NG Linum usitatissimum cDNA, mRNA sequence.", XP002793670, retrieved from EBI accession no. EM_EST:JG089421 Database accession no. JG089421
- DATABASE EMBL [Online] 13 March 2007 (2007-03-13), "1095458050015 Global-Ocean-Sampling_GS-26-01-01-1P3-1P6K B marine metagenome genomic clone 1061005667221 5', genomic survey sequence.", XP002793671, retrieved from EBI accession no. EM_GSS:EJ067028 Database accession no. EJ067028

## Description

### BACKGROUND

### Field

Despite numerous disadvantages to using methanol in protein expression, methanol inducible promoters, such as the *AOX1* promoter (*P_{AOX1}*), have been widely used in *Komagataella* yeast expression systems for protein expression.

### Description of the Related Art

*Komagataella phaffii* is a successful system for the production of a wide variety of recombinant proteins that are not native to the *Komagataella* cell. Several factors have contributed to its success as a protein manufacturing system, some of which include: (1) a promoter derived from the alcohol oxidase I (*AOX1*) gene of *K. phaffii* that is well suited for controlled expression of foreign genes; (2) similarity of techniques needed for the molecular genetic manipulation of *K. phaffii* to those of S. *cerevisiae,* which are well established ; (3) the strong preference of *K. phaffii* for respiratory growth, which is a key physiological trait that facilitates its culturing at high-cell densities relative to fermentative yeasts; and (4) the knowledge base on the *Komagataella* system as described in numerous recent publications. Furthermore, the genome of several *K. phaffii* species have been sequenced, which allows facilitated studies of the RNA and protein expression pathways. The culturing condition of *K. phaffii* is also relatively easy, as the cells can grow in a high density culture with high levels of proteins being expressed at the intra- and extra-cellular level.

*K. phaffii* is a single-celled microorganism that is easy to manipulate and culture. *K. phaffii* is a eukaryote capable of many of the post-translational modifications performed by higher eukaryotic cells such as proteolytic processing, folding, disulfide bond formation and glycosylation. Thus, the system may help to avoid loss of proteins that may end up as inactive inclusion bodies in bacterial systems, as bacterial systems lack methods of post-translation modifications. Foreign proteins requiring post-translational modification may be produced as biologically active molecules in *K. phaffii.* Additionally, the *K. phaffii* system has been shown to give higher expression levels of protein than many bacterial systems.

The ability of *K. phaffii* to utilize methanol as a sole source of carbon and energy was discovered in the 1970s. There are two alcohol oxidase genes *AOX1* and AOX2 which have strongly inducible promoters, the AOX promoters. These genes allow *Komagataella* to use methanol as a carbon and energy source. For example, the *AOX1* protein is produced in response to depletion of some carbon sources, such as glucose, and the presence of methanol. In some cases, the gene encoding a desired heterologous protein can be introduced under the control of the *AOX1* promoter, which means that gene expression and subsequent protein expression may be induced by the addition of methanol. As methanol could be synthesized from natural gas, methane, there was an interest in using these organisms for generating yeast biomass or single cell protein (SCP) to be marketed primarily as a high protein animal feed. During the 1970s, media and methods for growing *K. phaffii* on methanol in continuous culture at high cell densities (<130 g/l dry cell weight) were developed. However, during this same period, the cost of methane increased dramatically due to the oil crisis. Thus, the SCP process was never economically competitive for protein production.

Methods were then developed in the 1980's to produce *K. phaffii* as a heterologous gene expression system. The *AOX1* gene (and its promoter) was isolated and vectors, strains and methods for molecular genetic manipulation of *K. phaffii* were developed. The combination of strong regulated expression under control of the *AOX1* promoter along with the fermentation media and methods developed for the SCP process resulted in high levels of foreign proteins in *K. phaffii.*

Recombinant protein expression in *K. phaffii* may be driven by the promoter *AOX1* and induced by methanol and repressed by other carbon sources such as glucose, glycerol and ethanol. This induction and repression feature functions as a switch which turns recombinant protein expression on and off under different culture conditions. This switch is advantageous when expressing proteins that are toxic towards the host cell and towards cell growth. However, there are several limitations with this system. As the *AOX1* system requires methanol, the toxic and flammable material may require special handling and protocols. Additionally, hydrogen peroxide (H2O2) may be produced from methanol metabolism, which may also result in the degradation of recombinant proteins by the produced free radicals. The nature of methanol induction also limits where the manufacture location may be, and in some circumstances, may require long fermentation times and high biomass production. The production cost is considered to be high for a traditional *Komagataella* system (methanol inducible system).

As such, methods to identify promoters that drive protein expression independently of methanol that work as well as or better than methanol inducible promoters, are sought. A promoter that may drive protein expression of a protein independently of methanol in yeast may reduce the protein production cost and fermentation time. Additionally, there would be no need for food grade methanol in the process, thus allowing an easy and robust fermentation method for products such as edible and medical products. Thus, a promoter system for production of protein without methanol induction, or a constitutive promoter system would be advantageous for the production of recombinant proteins in the *K. phaffii* system.

### SUMMARY

In a first aspect, a method for identifying promoters that drive protein expression independently of methanol and are useful in driving protein expression in yeast is provided. The method comprises fermenting yeast cells under at least one fermentation condition in the absence of methanol, collecting samples at different times during fermentation under the at least one fermentation condition, determining the relative mRNA levels associated with native yeast genes in the samples, identifying one or more of the native yeast genes associated with higher than average levels of mRNA and determining putative promoters associated with the higher than average levels of mRNA encoding the native yeast genes, making expression constructs, each construct comprising a putative promoter, and a gene encoding a marker protein, introducing the expression constructs into yeast cells, culturing the yeast cells comprising the expression constructs in the absence of methanol, determining marker protein expression by the cultured yeast cells and comparing marker protein expression driven by each of the putative promoters to identify promoters that are useful in driving protein expression independently of methanol or are useful in driving constitutive protein expression in yeast. In some embodiments, the promoter drives protein expression in yeast. In some embodiments, the fermenting is performed with at least two different fermentation conditions in the absence of methanol.

In some embodiments, the method further comprises comparing mRNA levels for those more highly expressed genes from the at least two different fermentation conditions to identify a subset of genes that are more highly expressed across different fermentation conditions. In some embodiments, the method further comprises sequencing nucleic acids associated with the subset of genes to identify putative promoters or comparing sequences with publically available sequences. In some embodiments, the yeast cells are a species of methylotrophic yeast. In some embodiments, the yeast cells are of the genus *Komagataella.* In some embodiments, the yeast cells are selected from the group consisting of *K. farinosa, K. anomala, K. heedii, K. guilliermondii, K. kluyveri, K. membranifaciens, K. norvegensis, K. ohmeri, K. pastoris, K. methanolic, K. phaffiii and K. subpelliclosa.* In some embodiments, the yeast cell is *K. phaffiii.* In some embodiments, the collecting step comprises collecting samples at 0 hours, 24 hours, 43 hours, 49 hours, 69 hours, 75 hours, 90 hours, 100 hours, 116 hours, 122 hours, 142, and 168 hours, or any time in between a range defined by any two aforementioned valued during fermentation.

In some embodiments, the different fermentation conditions comprise different media pH, varying from a pH of 4, 5, 6, 7, 8 or any pH in between a range defined by any two aforementioned values. In some embodiments, the different fermentation conditions comprise different concentrations of at least one carbon source. In some embodiments, the at least one carbon source is selected from a group consisting of corn syrup, dextrose, maltose, glucose, dextrin, glycerol, sorbitol, mannitol, lactic acid, acetate, xylose, or other partially hydrolyzed starches, and any mixtures thereof. In some embodiments, the concentrations of the at least one carbon source varies from 0.0 g/L, 0.5g/L, 1g/L, 2 g/L, 4 g/L, 6 g/L, 8 g/L, 10 g/L, 11 g/L, 12 g/L, 13 g/L, 14 g/L, 15 g/L, 16 g/L, 18 g/L, 20 g/L, 22 g/L, 24 g/L, 26 g/L, 28g/L , 30g/L or 60 g/L or any concentration within a range defined by any two aforementioned values. In some embodiments, the identified putative promoter in the expression construct is 50, 100, 200, 300, 400, 500, 600, 700, 800, 900, 1000, 1500, 2000, 3000, 4000 or 5000 bases upstream from a translational start site of the gene, or any number of bases in between a range defined by any two aforementioned values upstream from the start site of the highly expressed gene. In some embodiments, the marker protein is a recombinant protein, peptide or an enzyme. In some embodiments, the enzyme is lipase, amylase, xylanase, protease, glucoamylase, glucanase, mannanase. phytase, or cellulase. In some embodiments, marker protein expression by the cultured yeast cells is determined in a small scale expression, e.g. in a microtiter plate. In some embodiments, the promoters that performed independently of methanol identified as useful in driving protein expression in a microtiter plate, are further tested under fermentation conditions. In some embodiments, the method further comprises testing the enzyme for activity for determining proper folding of protein and quantitations. In some embodiments, the at least one carbon source is selected from a group consisting of corn syrup, dextrose, maltose, glucose, dextrin, glycerol, sorbitol, mannitol, lactic acid, acetate, xylose, or other partially hydrolyzed starches, and any mixtures thereof.

### BRIEF DESCRIPTION OF THE DRAWINGS

**Figure 1** is a flow chart detailing method steps for isolating and identifying promoters that may lead to expression of protein in the absence of methanol or promoters that are independent of methanol for their function.
**Figure 2** shows the analysis of precision RNA sequencing, used for the methods for isolating the identified promoters that drive protein expression independently of methanol. As shown is a graph of the number of unique sequenced transcripts versus the single end reads for 10-time points (0907Y1-3, 0907Y1-4, 0907Y1-5, 0907Y1-6, 0907Y1-7, 0907Y1-8, 0907Y1-9, 0907Y1-10, 0907Y1-11, and 0907Y1-12).
**Figure 3** shows the results of the method for identifying promoters that drive protein expression independently of methanol, in which the transcript levels of two genes, SD001 and SD002, were identified after performing an RNA sequencing expression profile and selecting those genes for which the transcript level was high at all tested time points.
**Figure 4A** shows a protein gel profile comparing the expression of a lipase under the influence of the AOX promoter (inducible by methanol), promoter SD001 (drives expression independently of methanol), and the promoter SD002 (drives expression independently of methanol).
**Figure 4B** shows the lipase activity assay of the lipase that was expressed under the influence of the promoters, SD001 and SD002.
**Figure 5** shows protein gel for lipase expression under the control of promoters that drive protein expression independent of methanol during fermentation according one embodiment, as compared to a lipase gold standard expression.
**Figure 6** shows the correlation between whole broth lipase yields in 4 different fermentation conditions compared to relative lipase activity from microtiter plate samples.
**Figure 7** shows the lipase yield over time in fermenters. The identified promoter, SD001, drives protein expression independently of methanol. As shown in the left panel is the whole broth yield of lipase that is expressed and under the control of the SD001 promoter. The right panel shows the whole broth yield of lipase that is expressed and under the control of the AOX promoter in methanol-induced conditions.
**Figure 8A, 8B, and 8C** show that the SD001 promoter, identified by isolation methods for promoters that drive protein expression independently of methanol, drives the expression of diverse enzymes, such as a xylanase and two amylases in microtiter plates, as evidenced by the two protein gel profiles.

### DETAILED DESCRIPTION

In the description that follows, the terms should be given their plain and ordinary meaning when read in light of the specification. One of skill in the art would understand the terms as used in view of the whole specification.

As used herein, "a" or "an" may mean one or more than one.

"About" as used herein when referring to a measurable value is meant to encompass variations of ±20% or ±10%, more preferably ±5%, even more preferably ±1%, and still more preferably ±0.1 % from the specified value.

"Methylotrophic yeast," as described herein, have their plain and ordinary meaning when read in light of the specification, and may include but is not limited to, for example, a limited number of yeast species that can use reduced one-carbon compounds such as methanol or methane, and multi-carbon compounds that contain no carbon bonds, such as dimethyl ether and dimethylamine. For example, these species can use methanol as the sole carbon and energy source for cell growth. Without being limiting, methylotrophs may include the Genus *Methanoscacina, Methylococcus capsulatus, Hansenula polymorpha, Candida boidinii, Komagataella pastoris* and *Komagataella phaffii,* for example. In the embodiments described herein, a promoter that drive protein expression independently of methanol is provided for protein expression in a methylotrophic yeast cell.

*"Komagataella phaffii,"* has their plain and ordinary meaning when read in light of the specification, and may include but is not limited to, for example, a species of methylotrophic yeast. *"Pichia phaffii"* may also refer to the colloquial name as it has officially been renamed *Komagataella phaffii* (for the GS115 strain used herein) or it may be also referred to as *Komagataella pastoris,* depending on which lineage it has.

*Komagataella* is widely used for protein expression using recombinant DNA techniques since its alcohol oxidase promoters were isolated and cloned. Hence it is used in biochemical and genetic research in academia and the biotechnical industry as it can express a wide range of diverse genes as compared to other microorganism such as *Psesudomonas, Bacillus,* and *Aspergillus.* Furthermore, the protein product is easier to purify and leads to a clean product. *Komagataella* is well suited for protein expression as it has a high growth rate and is able to grow on a simple, inexpensive medium. *K. phaffii* can grow in either shaker flasks or a fermenter, which makes it suitable for both small and large scale production. *K. phaffii* has two alcohol oxidase genes *AOX1* and *AOX2,* which are strongly inducible promoters. These genes allow *Komagataella* to use methanol as a carbon and energy source. The *AOX* promoters are induced by methanol and are repressed by glucose, for example. Often, the gene for a desired heterologous protein is introduced under the control of the *AOX1* promoter, which means that protein expression can be induced by the addition of methanol. In a popular expression vector, the desired protein is produced as a fusion product to the secretion signal of the α-mating factor from *Saccharomyces cerevisiae* (baker's yeast). This causes the protein to be secreted into the growth medium, which greatly facilitates subsequent protein purification. *Komagataella* also has advantages over S. *cerevisiae* as well. *Komagataella* can easily be grown in cell suspension in reasonably strong methanol solutions that would kill most other microorganisms, a system that is difficult to set up and maintain. As the protein yield from expression in a microbe is roughly equal to the product of the protein produced per cell and the number of cells, this makes *Komagataella* of great use when trying to produce large quantities of protein without expensive equipment. However, *Komagataella* may be unable to produce proteins for which the host may lack the proper chaperones. As such, *Komagataella* may be co-transformed with a nucleic acid or a gene that encodes a chaperone for proper protein folding.

"Chaperone protein," "molecular chaperones," or "chaperones" have their plain and ordinary meaning when read in light of the specification, and may include but is not limited to, for example, proteins that assist the covalent folding or unfolding and the assembly or disassembly of other macromolecular structures. Chaperones are present when the macromolecules perform their normal biological functions and have correctly completed the processes of folding and/or assembly. The chaperones are concerned primarily with protein folding. In some embodiments of the methods of isolating a promoter that drives protein expression independently of methanol, the method further comprises co-transforming a cell with a vector encoding a chaperone and a vector encoding a promoter, wherein the promoter drives protein expression independently of methanol and a gene encoding a recombinant protein, such as for example, an enzyme. In some embodiments, the chaperone is expressed with the enzyme, wherein the chaperone assists in the folding of the enzyme. In some embodiments, expression of a chaperone leads to a functional enzyme. In some embodiments, the chaperone is expressed with a recombinant protein. In some embodiments, the promotor produces constitutively and is independent on the presence of methanol.

The budding yeast of strain *K. phaffii,* can grow on methanol and has been widely used for over 30 years for heterologous protein expression. For example, over 70 products including therapeutic biologicals (mostly) and industrial enzymes have been produced using the *K. phaffii* system. Protein from the system may be either secreted (>16g/L) or produced for intracellular expression (> 20g/L). Most enzyme companies produce enzymes using a native host or homologous expression of the enzyme. However no native enzymes from *Komagataella* have been discovered for industrial use. Also appreciated by those skilled in the art, are methods for genome sequencing and molecular tools available for strain manipulation. Growth of the cells, fermentation and production process are also well developed as the system has a long history of safe use and is regulatory friendly. Methods of growth of a typical culture for protein expression can be appreciated by those of skill in the art. In the embodiments provided herein, *K. phaffii* is used as an expression host for the expression of protein that is independent of methanol.

"Nucleic acid" or "nucleic acid molecule" have their plain and ordinary meaning when read in light of the specification, and may include but is not limited to, for example, polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. Nucleic acids can be either single stranded or double stranded. In some embodiments, a nucleic acid sequence encoding a fusion protein or recombinant protein is provided. In some embodiments, the nucleic acid is RNA or DNA. In some embodiments, the nucleic acid comprises a promoter that is not inducible by methanol. In some embodiments, a cell comprising the nucleotide for protein expression, wherein the protein expression is independent of methanol, is provided.

"Coding for" or "encoding" are used herein, and have their plain and ordinary meaning when read in light of the specification, and may include but is not limited to, for example, the property of specific sequences of nucleotides in a polynucleotide, such as a gene, a cDNA, or an mRNA, to serve as templates for synthesis of other macromolecules such as a defined sequence of amino acids. Thus, a gene codes for a protein if transcription and translation of mRNA corresponding to that gene produces the protein in a cell or other biological system. In some embodiments, a vector comprises a nucleic acid encoding a protein, wherein the nucleic acid encoding the protein is under the influence of a promoter that drives protein expression independently of methanol.

A "nucleic acid sequence coding for a polypeptide" has their plain and ordinary meaning when read in light of the specification, and may include but is not limited to, for example, all nucleotide sequences that are degenerate versions of each other and that code for the same amino acid sequence.

"Vector," "Expression vector" or "construct" have their plain and ordinary meaning when read in light of the specification, and may include but is not limited to, for example, a nucleic acid used to introduce heterologous nucleic acids into a cell that has regulatory elements to provide expression of the heterologous nucleic acids in the cell. The vector, as described herein, is a nucleic acid molecule encoding a gene that is expressed in a host-cell. Typically, an expression vector comprises a transcription promoter, a gene, and a transcription terminator. Gene expression is usually placed under the control of a promoter, and such a gene is said to be "operably linked to" the promoter. Similarly, a regulatory element and a core promoter are operably linked if the regulatory element modulates the activity of the core promoter. Vectors include but are not limited to plasmid, minicircles, yeast, and viral genomes. Available commercial vectors are known to those of skill in the art. Commercial vectors are available from European Molecular Biology Laboratory and Atum, for example.

A "Promoter that drives protein expression independently of methanol," has its plain and ordinary meaning when read in light of the specification, and may include but is not limited to, for example, a promoter that may allow an increase in the expression of a specific gene in the absence of methanol.

"Constitutive promoter," has its plain and ordinary meaning when read in light of the specification, and may include but is not limited to, for example, a promoter that is active in most circumstances in the cell. In some embodiments, a method for identifying promoters useful in driving protein expression in yeast is provided. In some embodiments, the promoter drives protein expression independently of methanol. The method comprises: fermenting yeast cells under at least one fermentation condition in the absence of methanol, collecting samples at different times during fermentation under the different fermentation conditions, determining the relative mRNA levels associated with native yeast genes in the samples, identifying one or more of the native yeast genes associated with higher than average levels of mRNA and determining putative promoters associated with the higher than average levels of mRNA encoding the native yeast genes, making expression constructs, each construct comprising one of the identified putative promoters and a gene encoding a marker protein and introducing the expression constructs into yeast cells, culturing the yeast cells comprising the expression constructs in the absence of methanol, determining marker protein expression by the cultured yeast cells and comparing marker protein expression driven by each of the putative promoters to identify promoters that drives protein expression independently of methanol that are useful in driving protein expression in yeast. In some embodiments, the promoter drives protein expression in yeast. In some embodiments, the fermenting is performed with at least two fermentation conditions in the absence of methanol. In some embodiments, the method further comprises comparing mRNA levels for those more highly expressed genes from the at least two different fermentation conditions to identify a subset of genes that are more highly expressed across different fermentation conditions. In some embodiments, the method further comprises sequencing nucleic acids associated with the subset of genes to identify putative promoters or comparing sequences with publically available sequences. In some embodiments, the yeast cells are a species of methylotrophic yeast. In some embodiments, the yeast cells are of the genus Komagataella. In some embodiments, the promoter is a constitutive promoter that may drives expression in the absence of methanol.

"Protein production," "protein expression," have their plain and ordinary meaning when read in light of the specification, and may include but is not limited to, for example, the biotechnological process of generating a specific protein. It may be achieved by the manipulation of gene expression in an organism such that it expresses large amounts of a recombinant gene. Without being limiting, this may include the transcription of the recombinant DNA to messenger RNA (mRNA), the translation of mRNA into polypeptide chains, which are ultimately folded into functional proteins and may be targeted to specific subcellular or extracellular locations.

"Fusion proteins" or "chimeric proteins" have their plain and ordinary meaning when read in light of the specification, and may include but is not limited to, for example, proteins created through the joining of two or more genes that originally coded for separate proteins or portions of proteins. The fusion proteins can also be made up of specific protein domains from two or more separate proteins. Translation of this fusion gene can result in a single or multiple polypeptides with functional properties derived from each of the original proteins. Recombinant fusion proteins can be created artificially by recombinant DNA technology for use in biological research or therapeutics. Such methods for creating fusion proteins are known to those skilled in the art. Some fusion proteins combine whole peptides and therefore can contain all domains, especially functional domains, of the original proteins. However, other fusion proteins, especially those that are non-naturally occurring, combine only portions of coding sequences and therefore do not maintain the original functions of the parental genes that formed them. In some embodiments, a method for identifying promoters that drives protein expression independently of methanol in yeast is provided. In some embodiments, the promoter is useful in driving expression of a fusion protein.

"Promoter" has its plain and ordinary meaning when read in light of the specification, and may include but is not limited to, for example, a nucleotide sequence that directs the transcription of a structural gene. In some embodiments, a promoter is located in the 5' non-coding region of a gene, proximal to the transcriptional start site of a structural gene. Sequence elements within promoters that function in the initiation of transcription may also be characterized by consensus nucleotide sequences. These promoter elements include RNA polymerase binding sites, TATA sequences, CAAT sequences, differentiation-specific elements (DSEs; McGehee et al., Mol. Endocrinol. 7:551 (1993);), cyclic AMP response elements (CREs), serum response elements (SREs; Treisman, Seminars in Cancer Biol. 1:47 (1990) ) glucocorticoid response elements (GREs), and binding sites for other transcription factors, such as CRE/ATF (O'Reilly et al., J. Biol. Chem. 267:19938 (1992);), AP2 (Ye et al., J. Biol. Chem. 269:25728 (1994);), SP1, cAMP response element binding protein (CREB; Loeken, Gene Expr. 3:253 (1993) and octamer factors (see, in general, Watson et al., eds., Molecular Biology of the Gene, 4th ed. (The Benjamin/Cummings Publishing Company, Inc. 1987; ) and Lemaigre and Rousseau, Biochem. J. 303:1 (1994);). A promoter may be constitutively active, repressible or inducible. If a promoter is an inducible promoter, then the rate of transcription initiation increases in response to an inducing agent. In contrast, the rate of transcription initiation is not regulated by an inducing agent if the promoter is a constitutive promoter. Repressible promoters are also known. In some embodiments, a gene delivery polynucleotide or vector is provided. In some embodiments, the gene delivery polynucleotide comprises a promoter sequence. The promoter can be specific for bacterial, mammalian or yeast expression, for example. In some embodiments, wherein a nucleic acid encoding a protein of interest is provided, the nucleic acid further comprises a promoter sequence. In some embodiments, the promoter is specific for expression in yeast. In some embodiments, the promoter is a conditional, inducible or a constitutive promoter. In some embodiments, the promoter is a promoter that drives protein expression independently of methanol, wherein protein expression is performed in a methanol-free media. The promoters isolated herein may be inducible or constitutive and may drive protein expression in the absence of methanol.

"Conditional" or "Inducible" has their plain and ordinary meaning when read in light of the specification, and may include but is not limited to, for example, a nucleic acid construct that includes a promoter that provides for gene expression in the presence of an inducer and does not substantially provide for gene expression in the absence of the inducer. In some embodiments, the promoter is an inducible promoter. In some embodiments, the promoter is an inducible promoter for yeast protein expression.

"Regulatory element" has its plain and ordinary meaning when read in light of the specification, and may include but is not limited to, for example, a regulatory sequence, which is any DNA sequence that is responsible for the regulation of gene expression, such as promoters and operators. The regulatory element can be a segment of a nucleic acid molecule, which is capable of increasing or decreasing the expression of specific genes within an organism. In some alternatives described herein, the gene is under a control of a regulatory element.

"Host cell" has its plain and ordinary meaning when read in light of the specification, and may include but is not limited to, for example, a cell that is introduced with a nucleic acid or vector that encodes a protein or gene of interest. In some embodiments, the host cell is an isolated cell. In the embodiments, described herein, the host cell is a yeast cell. In some embodiments, the cell is a methylotroph yeast cell. In some embodiments, the yeast cell is of *Komagataella phaffii.* In some embodiments, a method for identifying promoters that drives protein expression independently of methanol in yeast is provided. The method comprises the steps of: fermenting yeast cells under at least one fermentation condition in the absence of methanol, collecting samples at different times during fermentation under the different fermentation conditions determining the relative mRNA levels associated with native yeast genes in the samples, identifying one or more of the native yeast genes associated with higher than average levels of mRNA and determining putative promoters associated with the higher than average levels of mRNA encoding the native yeast genes, making expression constructs, each construct comprising one of the identified putative promoters and a gene encoding a marker protein and introducing the expression constructs into yeast cells, culturing the yeast cells comprising the expression constructs in the absence of methanol, determining marker protein expression by the cultured yeast cells and comparing marker protein expression driven by each of the putative promoters to identify promoters that drives protein expression independently of methanol that are useful in driving protein expression in yeast. In some embodiments, the promoter drives protein expression in yeast. In some embodiments, the fermenting is performed with at least two fermentation conditions in the absence of methanol. In some embodiments, the method further comprises comparing mRNA levels for those more highly expressed genes from the at least two different fermentation conditions to identify a subset of genes that are more highly expressed across different fermentation conditions. In some embodiments, the method further comprises sequencing nucleic acids associated with the subset of genes to identify putative promoters or comparing sequences with publically available sequences. In some embodiments, the yeast cells are a species of methylotrophic yeast. In some embodiments, the yeast cells are of the genus Komagataella. In some embodiments, the fermenting of yeast cells is performed under at least two fermentation conditions. Thus the promoters are identified and confirmed as promoters that drives protein expression independently of methanol by their influence on a marker protein in an isolated host cell. In some embodiments, the isolated host cell is a yeast cell. In some embodiments, the isolated host cell is *Komagataella phaffii.*

The term "gene expression" refers to the biosynthesis of a gene product. For example, in the case of a gene encoding a structural protein, gene expression involves transcription of the gene into mRNA and translation of mRNA into the structural protein.

"Protein" has its plain and ordinary meaning when read in light of the specification, and may include but is not limited to, for example, a macromolecule comprising one or more polypeptide chains. A protein can also comprise non-peptide components, such as carbohydrate groups. Carbohydrates and other non-peptide post-translational modifications can be added to a protein by the cell in which the protein is produced, and will vary with the type of cell. Proteins are defined herein in terms of their amino acid backbone structures; substituents such as carbohydrate groups are generally not specified, but can be present nonetheless. In some embodiments, a gene delivery polynucleotide or vector, is provided for expression of protein in a *Komagataella* system, wherein the expression is driven independent of methanol. In some embodiments, the gene delivery polynucleotide or vector further comprises a sequence for at least one protein.

"Gene" has its plain and ordinary meaning when read in light of the specification, and may include but is not limited to, for example, the molecular unit of heredity of a living organism, describing some stretches of deoxyribonucleic acids (DNA) and ribonucleic acids (RNA) that code for a polypeptide or for an RNA chain that has a function in the organism, and can be a locatable region in the genome of an organism.

"RNA sequencing" has its plain and ordinary meaning when read in light of the specification, and may include but is not limited to, for example, to methods for revealing the presence and quantity of RNA in a biological sample at a given moment in time, RNA sequencing may also be referred to as whole transcriptome shotgun sequencing. RNA sequencing is used to analyze the continually changing cellular transcriptome. Specifically, RNA sequencing may facilitate the ability to look at alternative gene spliced transcripts, post-transcriptional modifications, gene fusion, mutations/SNPs and changes in gene expression over time, or differences in gene expression in different groups or treatments. In addition to mRNA transcript analysis, RNA sequencing can look at different populations of RNA to include total RNA, small RNA, such as miRNA, tRNA, and ribosomal profiling. RNA sequencing may also be used to determine exon/intron boundaries and verify or amend previously annotated 5' and 3' gene boundaries. In the embodiments, herein, RNA sequencing is used to analyze the relative mRNA level of native genes. The RNA sequencing may be used to elucidate changes in gene expression as well as for the identifying the promoter sequence that allows the upregulated transcription of an mRNA sequence

In the case of the yeast, *Komagataella,* the genes are transcribed and spliced to produce mRNA transcripts. The mRNA may be extracted, fragmented and copied into stable double stranded cDNA. The double stranded cDNA may be sequenced using high throughput short read sequencing methods. The sequences may then be aligned to a reference genome to reconstruct which genome regions are transcribed. The data may then be used to annotate what the expressed genes are and their relative expression levels. Thus quantification of mRNAs may be possible by this method. RNA sequencing may be used to locate upregulated mRNA transcripts and may be used to isolate and identify specific promoters that drives protein expression independently of methanol in the *Komagataella* system.

Genetic modification performed by transformation is described herein. "Transformation" refers to transferring genetic material, such as, for example, nucleic acids, PCT amplified nucleic acids, or synthetic DNA or RNA, to a cell. Common techniques employed for transferring genetic material may use viruses or viral vectors, electroporation, and/or chemical reagents to increase cell permeability. In some alternatives herein, the isolated host cell is transformed by electroporation. In some embodiments, the isolated host cell is transformed by exposure to alkali cations in the presence of a vector, plasmid or DNA.

Various transformation techniques have been developed and can be appreciated by one of skill in the art. Thus, gene transfer and expression methods are numerous but essentially function to introduce and express genetic material in yeast cells.

The yeast *Komagataella phaffii* has been widely used as a heterologous protein expression host. Strong inducible promoters derived from methanol utilization genes or constitutive glycolytic promoters are typically used to drive gene expression. Notably, genes involved in methanol utilization are not only repressed by the presence of glucose, but also by glycerol.

As described herein, methods to identify and isolate novel promoters that may drive protein expression independently of methanol to drive high heterologous expression in scale-relevant fermentation conditions in *Komagataella phaffii* are provided. The promoters may be identified using a newly developed scale-relevant fermentation process to lower the overall biomass and to reduce cost of the production of protein. Methods for identifying new promoters that drives protein expression independently of methanol would be helpful for allowing robust and efficient high throughput screening in *Komagataella.*

The *Komagataella* genes of promoters that may drive protein expression independently of methanol were identified by methods using different fermentation conditions in combination with RNA sequencing. Further tests were executed to show that the identified promoters can influence heterologous gene expression in a small scale expression, e.g., in microtiter plate using fermentation conditions described in several embodiments herein.

Some promoters for expression of genes in the absence of methanol have been previously described. For example, inducible promoters have previously been published for small molecule induction. Without being limiting, current promoters that induced independently of methanol include *SUC2, P_{CUP1}, P_{GAL1} P_{ADH},* for example. However, the inducers for these specific promoters can be expensive. In addition, carbon-source dependent promoters have also been published. These can rely on relatively expensive carbon sources and can also be repressed by glucose, such as *P_{ADH2}, GLK1, HXK2* and *P1S1,* for example. Likewise, constitutive promoters have also been described, such as the glyceraldehyde-3-phosphate dehydrogenase (GAP). (Weinhandl *et al.* 2014;).

A problem with such known systems of promoters that drives protein expression independently of methanol for *Komagataella* is that these promoters have a weaker activity compared to the methanol-inducible *AOX1* promoters under fermentation conditions. Previous studies have focused on strong promoters from shaker flask conditions, which might not correlate well to performance in scale-relevant or full-scale fermentation conditions. An ideal promoter would be strongly induced under scale-relevant fermentation conditions.

Thus, methods for finding alternatives to methanol induced promoters are commercially desired to enable robust processes of protein production, low-cost medium components, and lower levels of biomass.

Described herein are methods for isolating and identifying *Komagataella* native promoters that are capable of driving protein expression in a media that lacks methanol. The methods described in the embodiments herein, were used to identify several promoters that can perform as well as or better than a methanol inducible promoter. For example, using the methods of the embodiments herein, several putative promoters that may drive protein expression independently of methanol were identified. Traditionally, in earlier literature, promoters were identified under the conditions of a shaker flask in an incubator. Without being limiting, there are several methods for identifying a promoter, such as bioinformatics algorithms to look for promoter motifs present upstream of a gene of interest and in combination of testing regions that have a proposed promoter motif in an expression vector, DNAse footprinting methods, mining sequences upstream from transcription start sites (TSS), RNA sequencing data, ChIP-sequencing data and gene prediction algorithms. Other methods may also include creating fusions with a reporter gene to look for the activity or presence of the encoded protein under the promoter, such as lacZ or GFP. Methods of promoter identification also use medium and growth conditions that are significantly different than the conditions for product production.

For *Komagataella,* the typical fermentation process generates a high biomass which is not preferred for enzyme production. Promoters that were capable of driving protein expression independently of methanol were identified using a method developed in the embodiments herein for a reduced biomass fermentation process. During this process, samples of the culture were taken at several different time points during fermentation. An mRNA sequencing method was then applied to give an insight into the gene expression and regulation profile during the fermentation process. This novel method allowed the identification of up-regulated, down-regulated, and constitutive promoters.

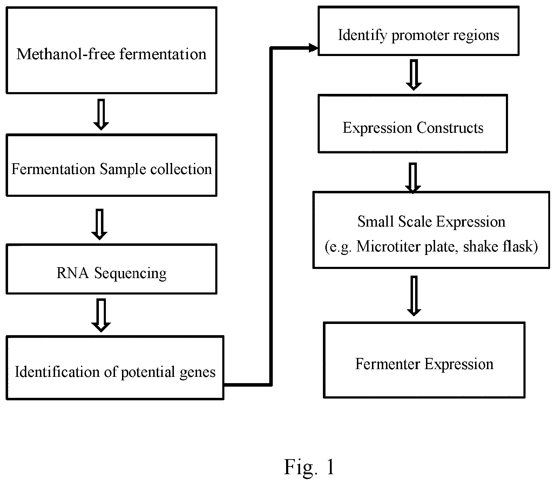

The methods for identifying promoters that may drive protein expression independently of methanol included the steps of methanol-free fermentation, fermentation sample collecting, RNA sequencing, identifying of potential genes that were expressed at a high level, identification of putative promoter regions, generation of expression constructs comprising putative promoters, and optionally using small scale expression, e.g., microtiter plate expression of test proteins and expression of the protein by fermenter. (See **Figure 1**).

The *Komagataella phaffii* used for the assay was strain GS115/ATCC 20864 with the pPIC9 vector integrated into the genome. This strain without the vector has a 9.4Mb sized genome with 5,073 predicted open reading frames (ORF). The vector adds 8 kb and at least 3 ORFs. In a liquid culture the cells were cultivated in shaker flasks at 30° C on a rotary shaker at 250 rpm with YPD media.

Either standard *Komagataella* media or media with reduced levels of phosphate was used. Phosphate reduced media was used in two of the strategies. The three feeding strategies that were assayed are:
1. Pulse feed of glucose -glucose feeding was controlled by dissolved oxygen (DO).
2. Glucose limited - a continuous rate of glucose was fed to the fermentation.
3. pH stat -glucose feeding was controlled by pH.

For the fermentation condition with pulse feed of glucose, the initial batch of *K. phaffii* cells were seeded with a batch culture, with a pH that was greater than a pH of 4.0. The fermentation was sampled two times a day for 140 hours, with the first-time point taken at 24 hours after the time of inoculation. The initial batch was supplemented with 15 g/L of glucose (corn syrup). The cells were given a 1 g/L pulse of glucose after initial glucose was used up controlled by DO.

For the fermentation condition for Glucose limited, the initial batch of *K. phaffii* cells were seeded with a batch culture with a pH that was greater than a pH of 4.0. The fermentation was sampled two times a day for 140 hours, with the first-time point taken at 24 hours after the time of inoculation. The initial batch was supplemented with 15 g/L of glucose (corn syrup). After initial glucose is all used up, the cells were given a 1G/L pulse of glucose until the OD₆₀₀ was more than 200, followed by constant feed of glucose.

For the fermentation condition with a pH stat control the initial batch of *K. phaffii* cells were seeded with a batch culture with a pH that was greater than a pH of 4.0. The fermentation was sampled two times a day for 140 hours, with the first-time point taken at 24 hours after the time of inoculation. The initial batch was supplemented with 15 g/L of glucose (corn syrup). After initial glucose is all used up, the cells were given a 1 g/L pulse of glucose until the OD₆₀₀ was more than 200, followed by pulse feed of glucose controlled at a pH greater than 6.0.

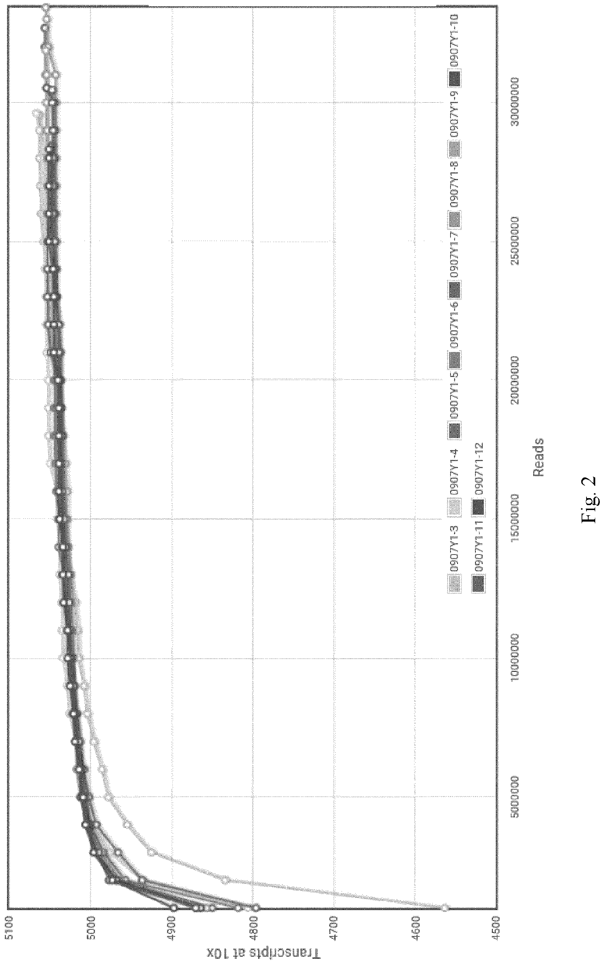

Samples were taken in triplicate two times a day for 140 hours, with the first-time point taken at 24 hours. The *K. phaffii* genome has been sequenced and the sequences of the expressed RNA are then identified and quantified from these time points. RNA sequencing was used to indicate the presence and the quantity of the RNA expressed in the cells at several time points. An RNA sequencing analysis of the total mRNA was performed using 75 base pair single-end reads to detect RNA expression in the samples of the three described fermentation conditions. Over 80% of the reads obtained were aligned to the genome (strain GS115/ATCC 20864) with the integrated pPIC9 vector sequence. The genome of GS115/ATCC 20864 has 5,073 predicted open reading frames and an additional 3 open reading frames are present in the pPIC9 vector. (See **Figure 2**).

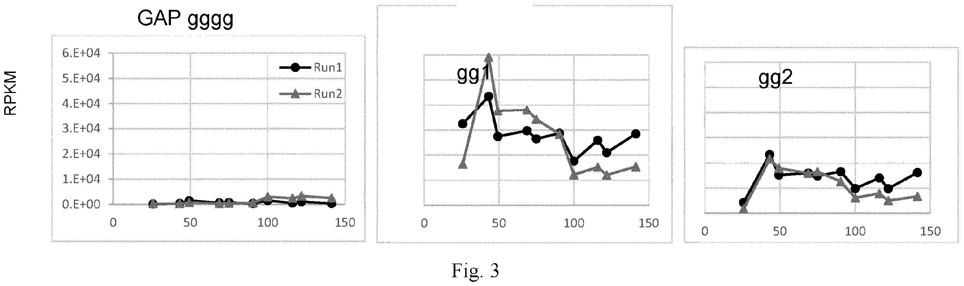

To obtain the RPKM (Reads per kilobase million), the count of the total reads corresponding to a single gene in a sample was divided by 1,000,000 to get RPM (Reads per million). The RPM was then divided by the length of the gene, in kilobases, to obtain the RPKM. For the gene ranking, each gene was ranked by the RPKM at each time point. The rankings for all the time points and biological replicates were then summed. The top genes from each fermentation condition were further analyzed to identify induced or constitutively high accumulated transcripts The two expressed genes were identified as SD001 and SD002. As shown in Figure 3, the SD001 gene had, on average, 2-10 times as much mRNA as the SD002 and GAP genes (the GAP promoter (glyceraldehyde-3-phosphate dehydrogenase) is commonly-used for constitutive expression of heterologous genes in *Komagataella* and is used as a control here).

Expression vectors were then constructed to test the influence of the putative promoter regions upstream of the SD001 gene and SD002 gene in expressing a reporter protein (lipase). Vectors for lipase expression were constructed with either the SD001 putative promoter or the SD002 putative promoter placed immediately upstream of the translational start site of a gene encoding a lipase (Vectors: SD002-promoter- lipase and SD001-promoter-lipase).

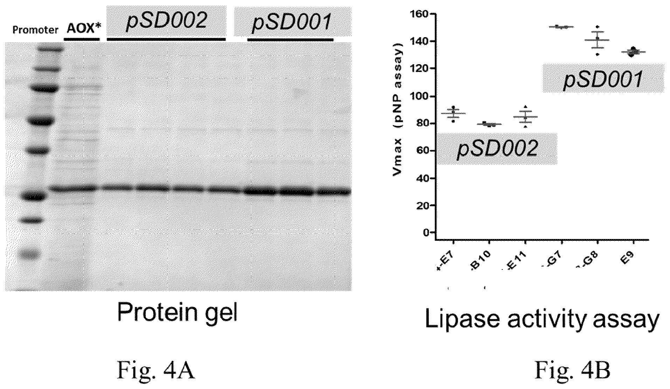

*Komagataella* cells were then transformed with either the construct, SD002-promoter-lipase or SD001-promoter-lipase, to test the ability of the putative promoters to drive lipase expression in a methanol-free environment. *Komagataella* cells transformed with the vector expressing lipase under control of an *AOX1* promoter was used as a control (methanol induction). The *Komagataella* containing the new expression constructs were grown in micro titer plates. As shown in **Figure 4A****,** the protein gel demonstrated that both promoters (SD001 and SD002) were functional for driving lipase expression in the absence of methanol, when compared to the *AOX1* promoter control vector, in which the *AOX1* was induced with methanol. The lipase activity assay also demonstrated that the lipases expressed under control of the putative promoter genes were produced as active protein (**See** **Figure 4B**). Because the lipase protein produced from either promoter is the same protein, the activity assay results can be used to quantify the relative amount of active lipase produced from the two promoters. The SD001 promoter was thus shown to drive the expression of twice the amount of lipase as compared to the SD002 promoter.

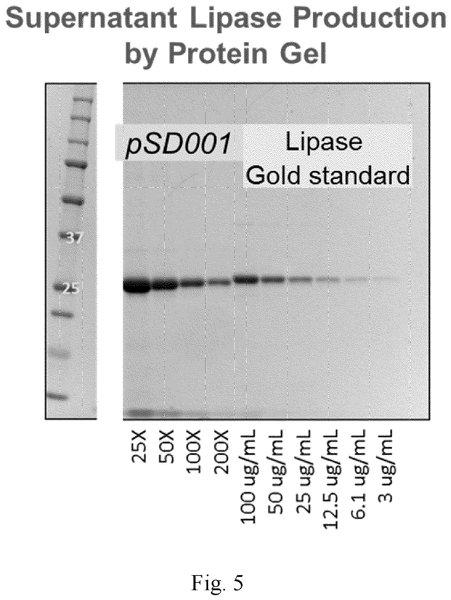

Lipase expression was also assayed under fermentation conditions using methanol-free conditions. *Komagataella* cells used for the assay included cells transformed with the vectors SD001-promoter-lipase. Figure 5 shows the protein gel for the Lipase production under the control of the SD001 promoter as compared to a lipase gold standard.

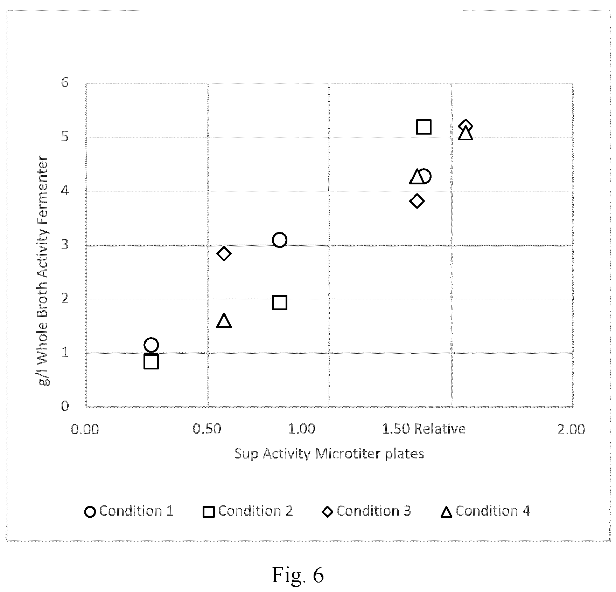

Six transformants with varying microtiter plate activities were tested in four different fermentation conditions. The six transformants were different individual transformants (colonies) transformed with the same vector. The transformants were screened in a small-scale batch for relative activity and then put into the fermenters. Relative activity in fermenters was then compared to the small-scale screening, showing good correlation between microtiter plate activity and fermentation yields. (**See** **Figure 6**).

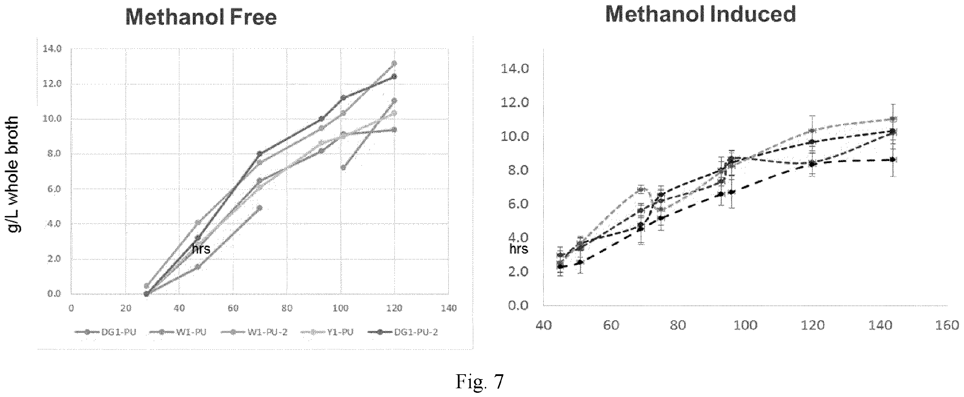

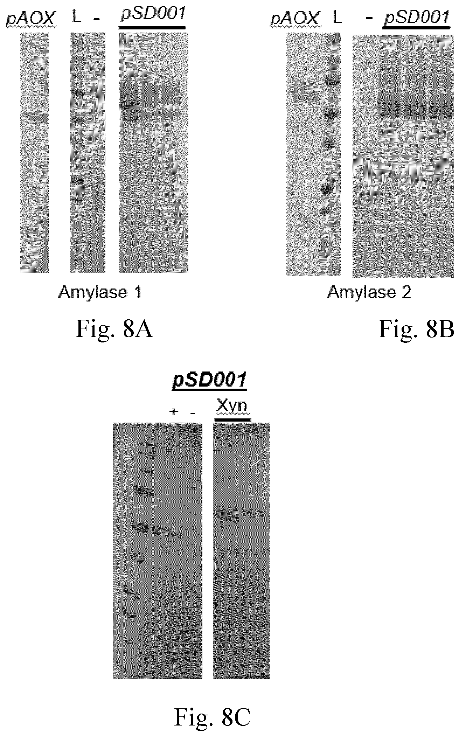

*Komagataella* cells transformed with vectors that included putative promoters that drive expression independently of methanol were also tested for their ability to drive expression of lipase over several fermentation runs (**See** **Figure 7**). The SD001 methanol-free promoter was shown to exhibit a higher induction activity in comparison to the AOX promoter. As shown, the lipase titer under the control of a SD001 promoter was shown to produce higher level compared under the control of the AOX promoter. Additionally, expression under the SD001 promoter increased in a linear fashion up from 0 to 120 hours of expression. As shown in **Figure 8A, 8B, and 8C****,** the identified promoters drive the expression of different classes of proteins (e.g. enzymes such as xylanase and amylases).

In conclusion, the new promoters identified by RNA sequencing showed expression of proteins in the absence of methanol in *Komagataella phaffii* in scale relevant fermentation conditions. The two promoters, the SD001 gene and the SD002 gene were validated for heterologous expression and were used to correlate expression between microtiter plates and fermenters, which also included scale-relevant conditions. The SD001 promoter was shown to be superior in protein expression when compared to the SD002 promoter and was shown to be capable of driving expression of multiple enzymes. The SD001 promoter was shown to be a stronger promoter than the methanol inducible promoter and thus has greater potential than the methanol inducible promoter for commercial production. Therefore, methods for isolating the promoters that drive protein expression independently of methanol, are described in the embodiments herein. Also described herein are the methods of testing the promoters for driving the expression of proteins such as amylase and lipase.

A method for identifying promoters that drive protein expression independently of methanol and are useful in driving protein expression in yeast is provided. The method comprises the following steps: fermenting yeast cells under at least one fermentation condition in the absence of methanol, collecting samples at different times during fermentation under the at least one fermentation condition, determining the relative mRNA levels associated with native yeast genes in the samples, identifying one or more of the native yeast genes associated with higher than average levels of mRNA and determining putative promoters associated with the higher than average levels of mRNA encoding the native yeast genes, making expression constructs, each construct comprising one of the identified putative promoters and a gene encoding a marker protein, and introducing the expression constructs into yeast cells, culturing the yeast cells comprising the expression constructs in the absence of methanol, determining marker protein expression by the cultured yeast cells, and comparing marker protein expression driven by each of the putative promoters to identify promoters that drive protein expression or constitutive protein expression independently of methanol and are useful in driving protein expression in yeast. In some embodiments, the promoter drives protein expression in yeast. In some embodiments, the fermenting is performed with at least two fermentation conditions in the absence of methanol. In some embodiments, the method further comprises comparing mRNA levels for those more highly expressed genes from the at least two different fermentation conditions to identify a subset of genes that are more highly expressed across different fermentation conditions. In some embodiments, the method further comprises sequencing nucleic acids associated with the subset of genes to identify putative promoters or comparing sequences with publically available sequences. In some embodiments, the yeast cells are a species of methylotrophic yeast. In some embodiments, the yeast cells are of the genus *Komagataella.* In some embodiments, the yeast cells consist of a group selected from *K. farinosa, K. anomala, K. heedii, K. guilliermondii, K. kluyveri, K. membranifaciens, K. norvegensis, K. ohmeri, K. pastoris, K. methanolic, K. phaffiii* and *K. subpelliclosa.* In some embodiments, the yeast cell is *K. pastoris.* In some embodiments, the yeast cell is *K. phaffiii.* In some embodiments, the collecting step comprises collecting samples at 0 hours, 24 hours, 43 hours, 49 hours, 69 hours, 75 hours, 90 hours, 100 hours, 116 hours, 122 hours, 142, and 168 hours, or any time in between a range defined by any two aforementioned valued during fermentation. In some embodiments, the different fermentation conditions comprise different media pH, varying from a pH of 4, 5, 6, 7, 8 or any pH in between a range defined by any two aforementioned values. In some embodiments, the different fermentation conditions comprise different concentrations of at least one carbon source. In some embodiments, the at least one carbon source is selected from a group consisting of corn syrup, dextrose, maltose, glucose, dextrin, glycerol, sorbitol, mannitol, lactic acid, acetate, xylose, or other partially hydrolyzed starches, and any mixtures thereof. In some embodiments, the at least one carbon source varies from 0.0 g/L, 0.5g/L, 1g/L, 2 g/L, 4 g/L, 6 g/L, 8 g/L, 10 g/L, 11 g/L, 12 g/L, 13 g/L, 14 g/L, 15 g/L, 16 g/L, 18 g/L, 20 g/L, 22 g/L, 24 g/L, 26 g/L, 28g/L , 30g/L or 60 g/L or any concentration within a range defined by any two aforementioned values. In some embodiments, the identified putative promoter in the expression construct is 50, 100, 200, 300, 400, 500, 600, 700, 800, 900, 1000, 1500, 2000, 3000, 4000 or 5000 bases upstream from a translational start site of the gene, or any number of bases in between a range defined by any two aforementioned values upstream from the start site of the highly expressed gene. In some embodiments, the marker protein is a protein, peptide, or an enzyme. In some embodiments, the enzyme is lipase, amylase, xylanase, protease, glucoamylase, glucanase, mannanase, phytase, or cellulase. In some embodiments, the method further comprises testing the enzyme for activity for determining proper folding of protein.

In some embodiments, the method further comprises testing the identified promoter that drives expression of protein independently of methanol, wherein the testing comprises construction of truncated versions of the promoter that drives expression of protein independently of methanol and ligating the truncated versions of the promoters that drives expression of protein independently of methanol to a gene encoding a marker protein, and testing expression of the genes under the control of the truncated versions of the promoter that drives expression of protein independently of methanol in a cell.

### Chaperone proteins

In some embodiments, the method further comprises testing the protein for enzyme activity or fluorescence. In some embodiments, wherein the protein lacks enzymatic activity or fluorescence, a host cell is transformed with the expression construct and a vector encoding a chaperone protein. The host cell with the expression construct and vector encoding the chaperone protein is then grown under fermentation conditions that allows expression of the enzyme or fluorescent protein. The enzyme or fluorescent protein is then tested for enzymatic activity or assayed for fluorescence. It is expected that a chaperone may assist in the proper folding of the enzyme or the fluorescent protein.

Sequences:
*pSD001-*SEQ ID NO: 1
p*SD002*-SEQ ID NO: 2

## Claims

1. A method for identifying promoters that are useful in driving protein expression in yeast, independently of methanol, the method comprising:
fermenting yeast cells under at least one fermentation condition in the absence of methanol;
collecting samples at different times during fermentation under the at least one fermentation condition;
determining the relative mRNA levels associated with native yeast genes in the samples;
identifying one or more of the native yeast genes associated with higher than average levels of mRNA;
determining putative promoters associated with the higher than average levels of mRNA encoding the native yeast genes;
making expression constructs, each construct comprising a putative promoter, and a gene encoding a marker protein;
introducing the expression constructs into yeast cells;
culturing the yeast cells comprising the expression constructs in the absence of methanol;
determining marker protein expression by the cultured yeast cells; and
comparing marker protein expression driven by each of the putative promoters to identify promoters that are useful in driving protein expression independently of methanol in yeast or promoters that are useful in driving constitutive or inducible protein expression in yeast.

2. The method of Claim 1, wherein the identified promoter drives protein expression in yeast.

3. The method of Claim 1 or 2, wherein the fermenting is performed with at least two different fermentation conditions in the absence of methanol.

4. The method of Claim 3, wherein the method further comprises comparing mRNA levels for those more highly expressed genes from the at least two different fermentation conditions to identify a subset of genes that are more highly expressed across different fermentation conditions.

5. The method of any one of Claims 1-4, wherein the method further comprises sequencing nucleic acids associated with the subset of genes to identify putative promoters or comparing sequences with publically available sequences.

6. The method of any one of Claims 1-5, wherein the yeast cells are a species of methylotrophic yeast.

7. The method of any one of Claims 1-6, wherein the yeast cells are of the genus *Komagataella.*

8. The method of any one of Claims 1-7, wherein the yeast cells are selected from the group consisting of *K. farinosa, K. anomala, K. heedii, K. guilliermondii, K. kluyveri, K. membranifaciens, K. norvegensis, K. ohmeri, K. pastoris, K. methanolic, K. phaffiii* and *K. subpelliclosa.*

9. The method of any one of Claims 1-8, wherein the yeast cell is *K. phaffiii.*

10. The method of any one of Claims 1-9, wherein the collecting step comprises collecting samples at 0 hours, 24 hours, 43 hours, 49 hours, 69 hours, 75 hours, 90 hours, 100 hours, 116 hours, 122 hours, 142, and 168 hours, or any time in between a range defined by any two aforementioned valued during fermentation.

11. The method of any one of Claims 1-10, wherein the different fermentation conditions comprise different media pH, varying from a pH of 4, 5, 6, 7, 8 or any pH in between a range defined by any two aforementioned values.

12. The method of any one of Claims 1-11, wherein the different fermentation conditions comprise different concentrations of at least one carbon source.

13. The method of Claim 12, wherein the at least one carbon source is selected from a group consisting of corn syrup, dextrose, maltose, glucose, dextrin, glycerol, sorbitol, mannitol, lactic acid, acetate, xylose, or other partially hydrolysed starches, and any mixtures thereof.

14. The method of Claim 13, wherein the concentration of the at least one carbon source varies from 0.0 g/L, 0.5g/L, 1g/L, 2 g/L, 4 g/L, 6 g/L, 8 g/L, 10 g/L, 11 g/L, 12 g/L, 13 g/L, 14 g/L, 15 g/L, 16 g/L, 18 g/L, 20 g/L, 22 g/L, 24 g/L, 26 g/L, 28g/L , 30g/L or 60 g/L or any concentration within a range defined by any two aforementioned values.

15. The method of any one of Claims 1-14, wherein the identified putative promoter in the expression construct is 50, 100, 200, 300, 400, 500, 600, 700, 800, 900, 1000, 1500, 2000, 3000, 4000 or 5000 bases upstream from a translational start site of the gene, or any number of bases in between a range defined by any two aforementioned values upstream from the start site of the highly expressed gene

16. The method of any one of Claims 1-15, wherein the marker protein is a protein, peptide or an enzyme.

17. The method of Claim 16, wherein the enzyme is lipase, amylase, xylanase, protease, glucoamylase, glucanase, mannanase, phytase, or cellulase.

18. The method of Claim 17, wherein the method further comprises testing the enzyme for activity for determining proper folding of protein.

## Patentansprüche

1. Verfahren zur Identifizierung von Promotoren, die sich zum Treiben der Proteinexpression in Hefe eignen, unabhängig von Methanol, wobei das Verfahren Folgendes umfasst:
Fermentieren von Hefezellen unter wenigstens einer Fermentationsbedingung in Abwesenheit von Methanol;
Entnehmen von Proben zu unterschiedlichen Zeitpunkten während der Fermentation unter der wenigstens einen Fermentationsbedingung;
Bestimmen der relativen mRNA-Spiegel in Zusammenhang mit nativen Hefegenen in den Proben;
Identifizieren eines oder mehrerer der nativen Hefegene in Zusammenhang mit überdurchschnittlich hohen mRNA-Spiegeln;
Bestimmen putativer Promotoren in Zusammenhang mit den überdurchschnittlich hohen Spiegeln von mRNA, die die nativen Hefegene codiert;
Herstellen von Expressionskonstrukten, wobei jedes Konstrukt einen putativen Promotor und ein ein Markerprotein codierendes Gen umfasst;
Einführen der Expressionskonstrukte in Hefezellen;
Kultivieren der die Expressionskonstrukte umfassenden Hefezellen in Abwesenheit von Methanol;
Bestimmen der Markerproteinexpression durch die kultivierten Hefezellen; und
Vergleichen der von den putativen Promotoren jeweils getriebenen Markerproteinexpression zur Identifizierung von Promotoren, die sich zum Treiben der Proteinexpression unabhängig von Methanol in Hefe eignen, oder Promotoren, die sich zum Treiben von konstitutiver oder induzierbarer Proteinexpression in Hefe eignen.

2. Verfahren nach Anspruch 1, wobei der identifizierte Promotor Proteinexpression in Hefe treibt.

3. Verfahren nach Anspruch 1 oder 2, wobei das Fermentieren bei wenigstens zwei unterschiedlichen Fermentationsbedingungen in Abwesenheit von Methanol erfolgt.

4. Verfahren nach Anspruch 3, wobei das Verfahren ferner Vergleichen von mRNA-Spiegeln für diese stärker exprimierten Gene aus den wenigstens zwei unterschiedlichen Fermentationsbedingungen zur Identifizierung einer Untergruppe von Genen, die über unterschiedliche Fermentationsbedingungen hinweg stärker exprimiert werden, umfasst.

5. Verfahren nach einem der Ansprüche 1-4, wobei das Verfahren ferner Sequenzieren von Nukleinsäuren in Zusammenhang mit der Untergruppe von Genen zur Identifizierung putativer Promotoren oder Vergleichen von Sequenzen mit öffentlich zugänglichen Sequenzen umfasst.

6. Verfahren nach einem der Ansprüche 1-5, wobei es sich bei den Hefezellen um eine Spezies von methylotropher Hefe handelt.

7. Verfahren nach einem der Ansprüche 1-5, wobei die Hefezellen zur Gattung *Komagataella* gehören.

8. Verfahren nach einem der Ansprüche 1-7, wobei die Hefezellen ausgewählt sind aus der Gruppe bestehend aus *K. farinosa, K. anomala, K. heedii, K. guilliermondii, K. kluyveri, K. membranifaciens, K. norvegensis, K. ohmeri, K. pastoris, K. methanolic, K. phaffiii* und *K. subpelliclosa.*

9. Verfahren nach einem der Ansprüche 1-8, wobei es sich bei der Hefezelle um *K. phaffiii* handelt.

10. Verfahren nach einem der Ansprüche 1-9, wobei der Entnahmeschritt Entnehmen von Proben zum Zeitpunkt 0 Stunden, 24 Stunden, 43 Stunden, 49 Stunden, 69 Stunden, 75 Stunden, 90 Stunden, 100 Stunden, 116 Stunden, 122 Stunden, 142 und 168 Stunden oder zu irgendeinem Zeitpunkt innerhalb eines durch zwei beliebige obengenannte Werte definierten Bereichs umfasst.

11. Verfahren nach einem der Ansprüche 1-10, wobei die unterschiedlichen Fermentationsbedingungen unterschiedliche Medien-pH umfassen, die von einem pH von 4, 5, 6, 7, 8 oder einem beliebigen pH-Wert innerhalb eines durch zwei beliebige obengenannte Werte definierten Bereichs variieren.

12. Verfahren nach einem der Ansprüche 1-11, wobei die unterschiedlichen Fermentationsbedingungen unterschiedliche Konzentrationen wenigstens einer Kohlenstoffquelle umfassen.

13. Verfahren nach Anspruch 12, wobei die wenigstens eine Kohlenstoffquelle ausgewählt ist aus einer Gruppe bestehend aus Maissirup, Dextrose, Maltose, Glucose, Dextrin, Glycerin, Sorbit, Mannit, Milchsäure, Acetat, Xylose oder anderen teilhydrolysierten Stärken und beliebigen Gemischen davon.

14. Verfahren nach Anspruch 13, wobei die Konzentration der wenigstens einen Kohlenstoffquelle von 0,0 g/l, 0,5 g/l, 1 g/l, 2 g/l, 4 g/l, 6 g/l, 8 g/l, 10 g/l, 11 g/l, 12 g/l, 13 g/l, 14 g/l, 15 g/l, 16 g/l, 18 g/l, 20 g/l, 22 g/l, 24 g/l, 26 g/l, 28 g/l, 30 g/l oder 60 g/l oder einer beliebigen Konzentration innerhalb eines durch zwei beliebige obengenannte Werte definierten Bereichs variiert.

15. Verfahren nach einem der Ansprüche 1-14, wobei der identifizierte putative Promotor im Expressionskonstrukt 50, 100, 200, 300, 400, 500, 600, 700, 800, 900, 1000, 1500, 2000, 3000, 4000 oder 5000 Basen stromaufwärts von einer Translationsstartstelle des Gens oder eine beliebige Anzahl Basen innerhalb eines durch zwei beliebige obengenannte Werte definierten Bereichs stromaufwärts von der Startstelle des stark exprimierten Gens liegt.

16. Verfahren nach einem der Ansprüche 1-15, wobei es sich bei dem Markerprotein um ein Protein, Peptid oder ein Enzym handelt.

17. Verfahren nach Anspruch 16, wobei es sich bei dem Enzym um Lipase, Amylase, Xylanase, Protease, Glukoamylase, Glukanase, Mannanase, Phytase oder Cellulase handelt.

18. Verfahren nach Anspruch 17, wobei das Verfahren ferner Testen des Enzyms auf Aktivität zur Bestimmung korrekter Proteinfaltung umfasst.

## Revendications

1. Procédé pour l'identification de promoteurs qui sont utiles pour commander l'expression de protéines dans une levure, indépendamment de méthanol, le procédé comprenant :
la fermentation de cellules de levure dans au moins une condition de fermentation en l'absence de méthanol ;
la collecte d'échantillons à des temps différents pendant la fermentation dans l'au moins une condition de fermentation ;
la détermination des niveaux d'ARNm relatifs associés à des gènes de levure natifs dans les échantillons ;
l'identification d'un ou plusieurs parmi les gènes de levure natifs associés à des niveaux d'ARNm plus hauts que la moyenne ;
la détermination de promoteurs putatifs associés aux niveaux d'ARNm plus hauts que la moyenne codant pour les gènes de levure natifs ;
la préparation de constructions d'expression, chaque construction comprenant un promoteur putatif, et un gène codant pour une protéine marqueur ;
l'introduction des constructions d'expression dans des cellules de levure ;
la culture des cellules de levure comprenant les constructions d'expression en l'absence de méthanol ;
la détermination de l'expression de protéine marqueur par les cellules de levure cultivées ; et
la comparaison de l'expression de protéine marqueur commandée par chacun des promoteurs putatifs pour identifier des promoteurs qui sont utiles dans la commande de l'expression de protéines indépendamment de méthanol dans une levure ou des promoteurs qui sont utiles dans la commande de l'expression de protéines constitutives ou inductibles dans une levure.

2. Procédé selon la revendication 1, le promoteur identifié commandant l'expression de protéines dans une levure.

3. Procédé selon la revendication 1 ou 2, la fermentation étant réalisée avec au moins deux conditions de fermentation différentes en l'absence de méthanol.

4. Procédé selon la revendication 3, le procédé comprenant en outre la comparaison de niveaux d'ARNm pour les gènes plus fortement exprimés des au moins deux conditions de fermentation différentes pour identifier un sous-ensemble de gènes qui sont plus fortement exprimés dans différentes conditions de fermentation.

5. Procédé selon l'une quelconque des revendications 1 à 4, le procédé comprenant en outre le séquençage d'acides nucléiques associés au sous-ensemble de gènes pour identifier des promoteurs putatifs ou comparer des séquences avec des séquences disponibles publiquement.

6. Procédé selon l'une quelconque des revendications 1 à 5, des cellules de levure étant une espèce de levure méthylotrophique.

7. Procédé selon l'une quelconque des revendications 1 à 6, les cellules de levure étant du genre *Komagataella.*

8. Procédé selon l'une quelconque des revendications 1 à 7, les cellules de levure étant choisies dans le groupe constitué par *K. farinosa, K. anomala, K. heedii, K. guilliermondii, K. kluyveri, K. membranifaciens, K. norvegensis, K. ohmeri, K. pastoris, K. methanolic, K. phaffiii* et *K. subpelliclosa.*

9. Procédé selon l'une quelconque des revendications 1 à 8, la cellule de levure étant *K. phaffiii.*

10. Procédé selon l'une quelconque des revendications 1 à 9, l'étape de collecte comprenant la collecte d'échantillons à 0 heure, 24 heures, 43 heures, 49 heures, 69 heures, 75 heures, 90 heures, 100 heures, 116 heures, 122 heures, 142 et 168 heures, ou à un quelconque moment dans une plage définie par deux valeurs quelconques mentionnées précédemment pendant la fermentation.

11. Procédé selon l'une quelconque des revendications 1 à 10, les conditions de fermentation différentes comprenant un pH de milieu différent, variant d'un pH de 4, 5, 6, 7, 8 ou un quelconque pH dans une plage définie par deux valeurs quelconques mentionnées précédemment.

12. Procédé selon l'une quelconque des revendications 1 à 11, les conditions de fermentation différentes comprenant différentes concentrations d'au moins une source de carbone.

13. Procédé selon la revendication 12, l'au moins une source de carbone étant choisie dans un groupe constitué par le sirop de mal̈s, le dextrose, le maltose, le glucose, la dextrine, le glycérol, le sorbitol, le mannitol, l'acide lactique, un acétate, le xylose, ou d'autres amidons partiellement hydrolysés, et de quelconques mélanges correspondants.

14. Procédé selon la revendication 13, la concentration de l'au moins une force de carbone variant de 0,0 g/L, 0,5 g/L, 1 g/L, 2 g/L, 4 g/L, 6 g/L, 8 g/L, 10 g/L, 11 g/L, 12 g/L, 13 g/L, 14 g/L, 15 g/L, 16 g/L, 18 g/L, 20 g/L, 22 g/L, 24 g/L, 26 g/L, 28 g/L, 30 g/L ou 60 g/L ou une quelconque concentration à l'intérieur d'une plage définie par deux valeurs quelconques mentionnées précédemment.

15. Procédé selon l'une quelconque des revendications 1 à 14, le promoteur putatif identifié dans la construction d'expression étant 50, 100, 200, 300, 400, 500, 600, 700, 800, 900, 1 000, 1 500, 2 000, 3 000, 4 000 ou 5 000 bases en amont d'un site de démarrage translationnel du gène, ou un quelconque nombre de bases dans une plage définie par deux valeurs quelconques mentionnées précédemment en amont du site de démarrage du gène fortement exprimé.

16. Procédé selon l'une quelconque des revendications 1 à 15, la protéine marqueur étant une protéine, un peptide ou une enzyme.

17. Procédé selon la revendication 16, l'enzyme étant une lipase, une amylase, une xylanase, une protéase, une glucoamylase, une glucanase, une mannanase, une phythase ou une cellulase.

18. Procédé selon la revendication 17, le procédé comprenant en outre le test de l'activité de l'enzyme pour la détermination du repliement correct d'une protéine.


### SEQUENCE LISTING

<110> BASF SE Tan, Xuqiu Dodge, Corey Wong, Kevin Li Milan, Aileen Aboy Orchard, Samantha Cai, Ruorong
<120> Methods for Identifying Promoters for Protein Production in Yeast
<130> 170841US01
<160> 2
<170> PatentIn version 3.5
<210> 1
   <211> 1501
   <212> DNA
   <213> Artificial Sequence
<220>
   <223> Description of Artificial Sequence: Synthetic Oligonucleotide
<400> 1
   tccagtgtag cactaaaatc taatatcttc ggctttatac ttttttgttc atccgaaagc      60
   ttacgaacaa ttctttctcc tgttttattg tggatataga caatttcgtc agtttcttgg    120
   agagaagagt tatttccggt tttggctggc cctataaacg ggttcttgga tttggatcta    180
   gtaataaaaa tgtcactgtc attctcggag ctgaactttg tgttgtacga agatgggttg    240
   ttccactgtt ttgccagctc ttcattgatg attttcttag tgggtgttct tggaggttca    300
   cgttgcctat aatcttgacg ttcttcttca tcactatcga tgccatcaaa attaagcgtc    360
   cttattgcag gcttttgtga tttcaactgc aatccttcta tctcttcatc agagctttcg    420
   aactgaatac tatcactcaa aactggcgac attgcacatt tccgcaaacc atttcgggaa    480
   tctatgctag ctcttctaga cgataaagaa cgaccggaac caatacgggg ttgtgcaggt    540
   gggaataaat atgttggttt ggattcttga cgtgaagaag gtattctagt cgatgaagtg    600
   gttgataagg atatggcgtc actgagttgt tttcttttcc tatgttgcgg tgttgggtca    660
   ggagttaatt gattcacctc cataactctg gaatttcttg aatgtggggt tttcagatgg    720
   gcatctttct tgacggggtt gtgagtaacg gaggaacctg gtgtcttggg tgtgaacggt    780
   gtttgagcct gtacgcggtt acttctgggc ggagtactcg gagtcatgag agccattgat    840
   tagaaggtga atgagggagt caccactcta agcaaacaaa atgaggtcga agcaaaaaat    900
   aaagtaaagt agcacttctg gcaggttaga tcaaagagtg acgggagatt tgaagatggc    960
   tggtttttcc ttagtcttgg aagaggtttg tgtgggtatc agcgaatatt ccccgattag   1020
   gcaaattagt tgcattgaaa ttaacacgac atggtgattt gtggtaacaa atatctattg   1080
   gtggttggtg tgtgggtgta atagtggtcg tgtcatgatg atggtgttca ggtgttgtca   1140
   tagatcggtc ttcagtaaga gaaggaagct tggtgacgat cacagctatg atgtaataga   1200
   aattgctaag caattgtgag gtgtgatgta ttttgcagag caattgtgcg gtacaacggg   1260
   gtgttattgt cttcacaagg catttattgc gaatttcgta gttgaaagaa tattttagca   1320
   cagggtgctt gacccctatt gttgctcgct aaaccatgat tgctaaatga tgacatagca   1380
   atcactttac taagattgct ataaggacac ctttcttagt ataaatggac actcttttcc   1440
   cctgctaaac ttcttttatt tttcacactt aaacagttac aaaacacaaa cacaactaga   1500
   a                                                                   1501
<210> 2
   <211> 1501
   <212> DNA
   <213> Artificial Sequence
<220>
   <223> Description of Artificial Sequence: Synthetic Oligonucleotide
<400> 2
   gtgctaaaat ctgaggttta caagctgtga tgttccccta agatctcaca atcgaacaat      60
   cgcgaagcca atgcaagttg tttaagggga aacgactcac tattcctgaa attagtattc     120
   aaaacttggt ccggaagaac aatgaggcgg ccgttaaaat actcacgtaa acggtgtcta     180
   caagcgcatt aaaatccgtt tgaattcaag caaaagccac cagaggctta tgcttggtta     240
   tacccagcat tgacctttgg tatgagcatc tgaaaaacaa ccaggtgttg caaagttaaa     300
   catccttctt tgttcatata gaacccacta ttcatggtac tccccaatcg aatttcacat     360
   tctggttttg aaattacaca ccacgttagc ttataagatt tcatataact tattgatata     420
   cggtttccat tgttcgaata gttgaggttg tatgtaattc gattgaaggg gccatttttg      480
   tttcctactt ttcctgggag cttatccgat gcgcttcaaa gctggaattg taaatataga     540
   gaaaaagaag gatgttgttt tattcttgaa agagtataat tttacttcta gcaactctcc     600
   cacttcgctt gacttcattt atttcttggg cacataggcg tagtaatcta gaccaacaga     660
   taatttgccg gaatgatata gcgattggaa aatgaactga aattttttgc tgtctttcaa      720
   tttgacgggc agttcatcag tgaccgacca tataaatacg ttgagaatgt tattcttcct     780
   cgtagttgaa gtggcttcat aatttcagaa ctcaatagat aaactaggat gttttaaagc     840
   aattaatgct cacaagtaag gagcgactct cttgcttttc gaatactaaa agtatcgtcc     900
   caacccagaa aaaaagacct cttaactgca aaataaactc tatatatttc ttctaaaaca     960
   gtttcaggtt ggatagtatc gcattctcat cacttctaac tagtaggcca tgagatatat    1020
   taacgtttac ttgagttcta agttctccga attagatgca cagcacaaac aagattaggt    1080
   ttcacttggt acaaaatacg aacagagttt aaggtcgtaa tttcatttcg ttattgatcc    1140
   ccacaatcta ttcttatcac agtcatcaga tagtcgcgaa aaagcatgca gaaaaggggg    1200
   tcgtccctat ctaagttgta gcattacaac aaatatgact acactcagtg tcgcaatcgg    1260
   tatagccaac gctgcaaaat ggattctact gagaatggta tgatgatccc aggatcaatt    1320
   tcccaaaaat taaaaaaagt aaaataaaaa gcatcagata ttagggaggt ggtaagattg    1380
   ctctgcaagc gatcacgaga ttttaggttt tcctttatgt actatataaa gcgcagattg    1440
   gatgccgctt ttccctcctg ggctatgata atatagcgaa cgaaatacac gccaaaataa    1500
   a                                                                    1501